# EUROPÄISCHE PATENTANMELDUNG

(11) **EP 0 821 974 A1**
(43) Veröffentlichungstag der Anmeldung: **04.02.1998**
(21) Anmeldenummer: 96810513.0
(22) Anmeldetag: 02.08.1996
(51) Int. Cl.: A61M 5/158

(54) **Instrument zum rechtwinkligen Herausziehen einer Kanüle aus einem Port**

(71) Anmelder: Beracon AG, 4614 Hägendorf (CH)
(72) Erfinder: Mäder, Gerold, 4616 Kappel (CH); Wüthrich, Martin, 4652 Winznau (CH)
(74) Vertreter: BOVARD AG - Patentanwälte

(57) **Zusammenfassung**

Das Instrument (1) zum rechtwinkligen Herausziehen von Kanülen aus einem implantierten Kathetersystem umfasst einen Träger (11) mit einer am unteren Ende desselben befestigten Schaufel (13). In einem im unteren Bereich des Trägers (11) angeordneten Längsschlitz (14) ist ein Schieber (15) mit einer oberen Schaufel (17) verschiebbar vorgesehen. Die beiden Schaufeln (13, 17) sind in der Mitte mit je einem Schlitz (12, 19) versehen. Der Schieber ist im verbreiterten Teil (21) mit einem Loch (16) zum Einführen des vorderen Teils eines Fingers versehen. Im oberen Teil des Trägers (11) ist ein Loch (19) für den Daumen der rechten oder linken Hand des Pflegepersonals vorgesehen. In der unteren Position A des Schiebers (15) liegen die beiden Schaufeln (13, 17) aufeinander. In dieser Position wird mit den beiden Schaufeln zwischen Haut und Kanülenhalter (9) eingefahren. Mit der linken oder rechten Hand wird der Halter (9) für die Kanüle gehalten. Durch Anheben des Schiebers (15, 16, 21) in die Position B wird die Kanüle (2) rechtwinklig aus der Portmembrane (7) des implantierten Ports (4) gezogen. Wobei bisher beim Herausziehen des Halters (9) mit der Kanüle (2) Verletzungen auftraten, so kann mit dem Instrument (1) die Verletzungsgefahr minimalisiert oder gänzlich vermieden werden.

## Beschreibung

Die vorliegende Erfindung bezieht sich auf ein Instrument zum rechtwinkligen Herausziehen einer Kanüle aus einem Port gemäss dem Oberbegriff des Patentanspruches 1.

Zum Auffüllen des Reservoirs des Ports eines implantierten Kathetersystemes wird eine Kanüle durch die Membrane des Ports in das Reservoir eingeführt. Die Kanüle ist mit einem Schlauch mit der Injektionsspritze verbunden. Nach dem Auffüllen des Reservoirs wird die Kanüle vom Pflegepersonal von Hand herausgezogen. Dabei ergeben sich oftmals Verletzungen des Pflegepersonals sowie der Patienten. Es ist deshalb die Aufgabe der vorliegenden Erfindung, ein Instrument zum rechtwinkligen Herausziehen einer Kanüle aus einem Port zu schaffen, mit welchem Instrument Verletzungen minimalisiert oder gänzlich vermieden werden können. Dies wird erfindungsgemäss erzielt durch einen Träger mit einem unteren, mit dem Träger verbundenen Haltemittel und ein relativ zum Haltemittel verschiebbares Hebemittel, wobei im Träger und am Hebemittel Organe zum Festhalten des Instrumentes und zum Verschieben des Hebemittels vorgesehen sind.

Bevorzugte Ausführungsformen ergeben sich aus den abhängigen Patentansprüchen.

Im folgenden wird anhand der beiliegenden Zeichnung ein Ausführungsbeispiel der Erfindung sowie dessen Verwendung näher beschrieben. Es zeigen
Fig. 1 eine perspektivische Darstellung des Instrumentes zum Entfernen einer Kanüle aus einem ebenfalls perspektivisch gezeichneten, implantierten Kathetersystem
Fig. 2 eine Seitenansicht des Instrumentes
Fig. 3 eine Vorderansicht des Instrumentes
Fig. 4 eine Draufsicht auf das Instrument.

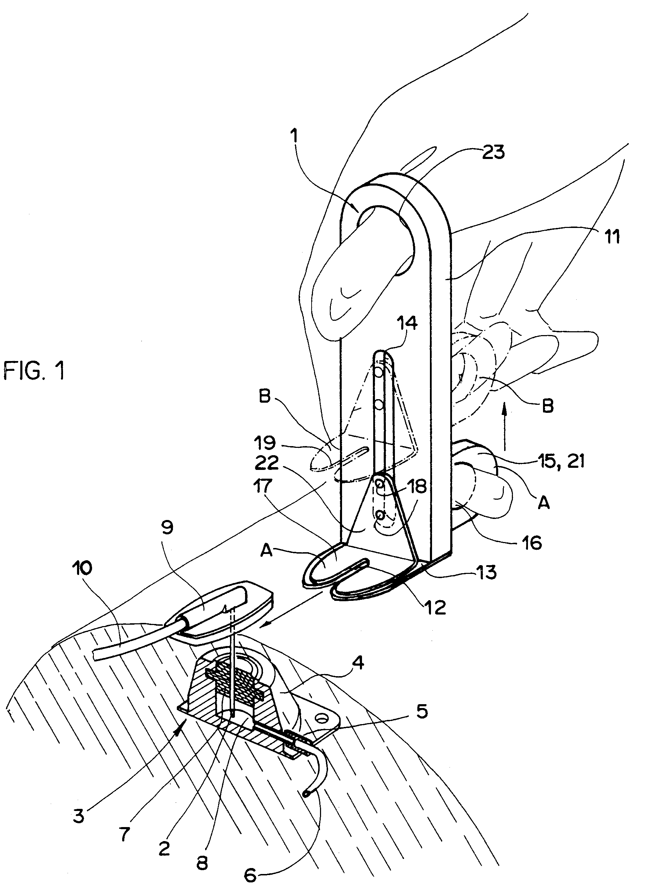

In Fig. 1 ist das Instrument 1 zum Entfernen einer Kanüle 2 aus einem im Körper implantierten Kathetersystem 3 sowie das Kathetersystem perspektivisch dargestellt. Das implantierte Kathetersystem 3 umfasst einen Port 4 zur Aufnahme der Injektionsflüssigkeit. Der Port 4 besitzt einen Anschluss 5 für den Venenkatheter 6. Im oberen Teil des Ports ist eine Portmembrane 7 aus Silikon eingelassen, welche ein Reservoir 8 zur Aufnahme der Injektionsflüssigkeit begrenzt. Ein Halter 9 für die Kanüle 2 ist mit einem Schlauch 10 zum Anschluss an eine nicht dargestellte Injektionsspritze verbunden. Die Kanüle 2 wird mit dem Halter 9 zum Nachfüllen des Reservoirs 8 im Port durch das Körpergewebe und die Portmembrane 7 gestossen. Beim Herausziehen der Kanüle 2 mit der Hand ergeben sich oftmals Verletzungen des Pflegepersonals sowie der Patienten. Mit dem Instrument 1 zum Herausziehen der Kanüle 2 rechtwinklig zur Portmembrane 7 können diese Verletzungen eliminiert werden. Das Instrument umfasst einen Träger 11, an dessen unterem Ende eine feste, mit einem Schlitz 12 versehene, plane Schaufel 13 mit Kerbnieten rechtwinklig zum Träger 11 befestigt ist. Der Schlitz 12 erstreckt sich in der Mitte der Schaufel 13. In einem sich im unteren Bereich des Trägers 11 befindlichen Längsschlitz 14 ist ein Schieber 15 geführt. Der Schieber 15 weist einen verbreiterten Teil 21 mit einem Fingerloch 16 auf. Der Schieber ist an der vorderen vom Fingerloch abgewandten Seite mit einer oberen, durch den Schieber 15 parallel zum Längsschlitz beweglichen, ebenfalls rechtwinklig zum Träger 11 angeordneten, planen Schaufel 17 versehen. Die obere Schaufel 17 ist ebenfalls mit einem sich in der Mitte der Schaufel erstreckenden Schlitz 19 versehen. Ein mit der Schaufel 17 verbundener Ansatz 22 ist mit zwei Kerbnieten 18 am Schieber 15 befestigt. Der verbreiterte Teil 21 und der Ansatz 22 verhindern ein Herausrutschen des Schiebers aus dem Längsschlitz 14. Im oberen Bereich des Trägers 11 befindet sich ein Loch 23 für den Daumen der rechten oder linken Hand des Pflegepersonals. Mit einem weiteren Finger der rechten oder linken Hand wird das Fingerloch 16 erfasst. Mit der linken oder rechten Hand wird der Halter 9 für die Kanüle 2 gehalten. In der Position A liegt die bewegliche Schaufel 17 auf der festen Schaufel 13 auf. Es wird mit den beiden Schaufeln zwischen der Haut und dem Halter 9 für die Kanüle eingefahren, wobei die Kanüle in die Schlitze 12 und 19 der Schaufeln gelangt. Durch Hochheben des Schiebers 15, 16, 21 mit der Schaufel 17 in die Position B wird der Halter 9 mit der Kanüle 2 rechtwinklig aus der Portmembrane 7 gezogen. Mit der unteren festen Schaufel 13 wird dabei auf den Port 4 gedrückt. Der Träger 11, der Schieber 15 und der verbreiterte Teil 21 sind aus sterilisierbarem Kunststoff POM gefertigt. Der Kunststoff wird im Spritzgussverfahren hergestellt. Die Schaufeln 13 und 17 sind aus elektropoliertem, rostfreiem Federstahl hergestellt. Die Schaufeln müssen so dünn gefertigt sein, damit man mit ihnen zwischen der Haut und dem Halter 9 für die Kanüle einfahren kann. Das Instrument 1 ist gratfrei und nicht scharfkantig hergestellt. Mit dem Instrument 1 wird jegliche Verletzungsgefahr minimalisiert oder gänzlich vermieden.

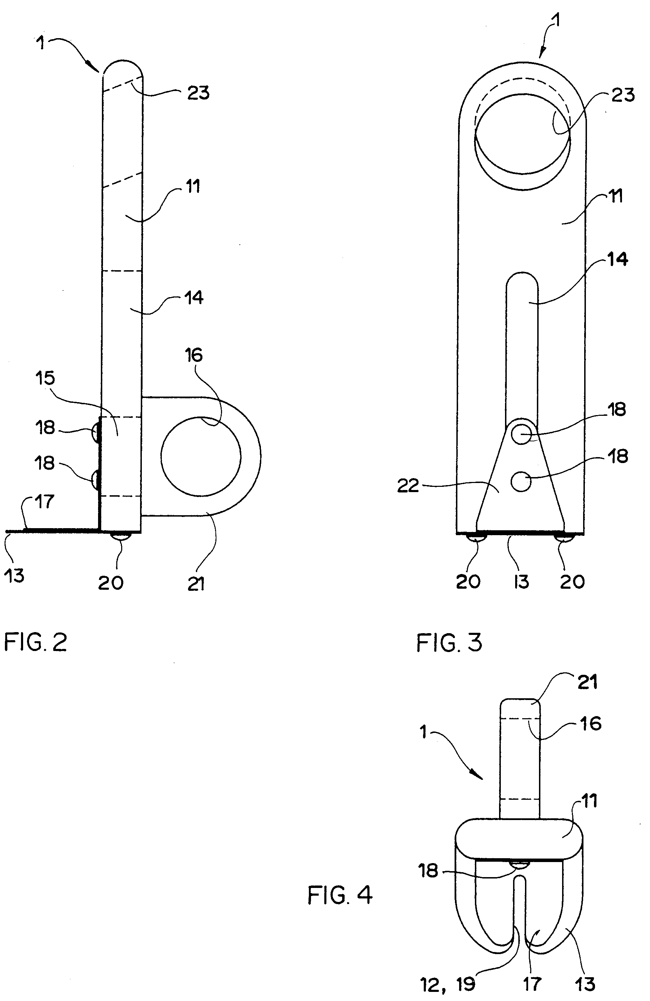

In Fig. 2 ist eine Seitenansicht des Instrumentes 1 dargestellt.

Fig. 3 zeigt eine Vorderansicht des Instrumentes. Aus dieser Figur sind noch die unteren Kerbnieten 20 zur Befestigung der unteren Schaufel am Träger 11 ersichtlich.

Fig. 4 zeigt eine Draufsicht auf das Instrument 1.

## Patentansprüche

1. Instrument (1) zum rechtwinkligen Herausziehen einer Kanüle aus einem Port, gekennzeichnet durch einen Träger (11) mit einem unteren, mit dem Träger verbundenen Haltemittel (13) und ein relativ zum Haltemittel (13) verschiebbares Hebemittel (17), wobei im Träger (11) und am Hebemittel (17) Organe (15, 16, 21, 23) zum Festhalten des Instrumentes (1) und zum Verschieben des Hebemittels (17) vorgesehen sind.

2. Instrument nach Patentanspruch 1, dadurch gekennzeichnet, dass das Haltemittel (13) und das Hebemittel (17) mit je einem Schlitz (12, 19) versehen sind, wobei sich die Schlitze überdecken, wenn das Haltemittel (13) und das Hebemittel (17) übereinander liegen.

3. Instrument nach Patentanspruch 1 oder 2, dadurch gekennzeichnet, dass das Haltemittel (13) und das Hebemittel (17) plan ausgebildet sind.

4. Instrument nach einem der vorangehenden Patentansprüche, dadurch gekennzeichnet, dass das Hebemittel (17) mit einem Schieber (15) verbunden ist, der in einem Längsschlitz (14) des Trägers (11) geführt ist.

5. Instrument nach Patentanspruch 4, dadurch gekennzeichnet, dass der Schieber (15) auf der dem Hebemittel (17) abgewandten Seite mit einem verbreiterten Teil (21) versehen ist, der eine Fingeröffnung (16) aufweist.

6. Instrument nach Patentanspruch 4 oder 5, dadurch gekennzeichnet, dass das Hebemitte (17) mit einem mit dem Schieber (15) verbundenen Ansatz (22) versehen ist.

7. Instrument nach einem der vorangehenden Patentansprüche, dadurch gekennzeichnet, dass der Träger (11) im oberen Bereich eine Fingeröffnung (23) aufweist.

8. Instrument nach einem der vorangehenden Patentansprüche, dadurch gekennzeichnet, dass der Träger (11), der Schieber (15) und der mit diesem verbundene, verbreiterte Teil (21) aus sterilisierbarem Kunststoff bestehen.

9. Instrument nach Patentanspruch 8, dadurch gekennzeichnet, dass als Kunststoff POM verwendet wird.

10. Instrument nach einem der Patentansprüche 1 bis 7, dadurch gekennzeichnet, dass der Träger (11), der Schieber (15) und der mit diesem verbundene, verbreiterte Teil (21) aus Aluminium bestehen.

11. Instrument nach einem der vorangehenden Patentansprüche, dadurch gekennzeichnet, dass das Haltemittel (13) und das Hebemittel (17) mit dem Ansatz (22) aus rostfreiem, elektropoliertem Federstahl bestehen.
